# EUROPÄISCHE PATENTANMELDUNG

(11) **EP 0 628 282 A1**
(43) Veröffentlichungstag der Anmeldung: **14.12.1994**
(21) Anmeldenummer: 94107971.7
(22) Anmeldetag: 25.05.1994
(51) Int. Cl.: A61B 5/14, A61B 17/32

(54) **Blutentnahmevorrichtung und Verfahren zu deren Herstellung**

(30) Priorität: 02.06.1993 DE 4318142
(71) Anmelder: Herbst, Richard, D-85386 Eching (DE)
(72) Erfinder: Herbst, Richard, D-85386 Eching (DE)
(74) Vertreter: Witte, Alexander, Dr.-Ing.

(57) **Zusammenfassung**

Eine Blutentnahmevorrichtung umfaßt ein Lanzettenteil (11) und ein Buchsenteil (12). Das Lanzettenteil (11) ist an seinem einen Ende mit einem Knopfteil (30) und an seinem anderen Ende mit einem vorstehenden Schneidenabschnitt (24) einer Lanzette (23) versehen. Das Buchsenteil (12) ist zur Aufnahme und zur axialen Führung des Lanzettenteils (11) ausgebildet. Es ist an seinem einen Ende mit einem Ringflansch (45) versehen, der in einer ersten Gebrauchsstellung der Vorrichtung mittels eines mechanisch überwindbaren Anschlages in einem ersten Abstand von dem Knopfteil (30) angeordnet ist, derart, daß sich der Schneidenabschnitt (24) innerhalb des Buchsenteils (12) befindet. Das Knopfteil (30) und der Ringflansch (45) sind unter Überwindung des Anschlages schlagartig in eine zweite Gebrauchsstellung aneinander mit einem zweiten, geringeren Abstand voneinander überführbar, so daß der Schneidenabschnitt (24) aus dem anderen Ende des Buchsenteils (12) vorsteht. Der mechanisch überwindbare Anschlag ist als Sollbruchstelle ausgebildet. Ferner sind zum wahlweisen Einstellen des ersten Abstandes mindestens zwei Abstandshalter vorgesehen, die alternativ in der zweiten Gebrauchsstellung einen unterschiedlichen zweiten Abstand herstellen. Weiterhin ist an das Lanzettenteil ein Schutzelement angesetzt, das den Schneidenabschnitt umhüllt und von diesem durch Fingerkraft lösbar ist, wobei das Schutzelement vor der Benutzung der Vorrichtung aus dem anderen Ende des Buchsenteils (12) vorsteht und dort mit Fingern ergreifbar ist. Schließlich wird ein Verfahren zur Herstellung einer derartigen Blutentnahmevorrichtung beschrieben.

## Beschreibung

Die Erfindung betrifft eine Blutentnahmevorrichtung mit einem Lanzettenteil und einem Buchsenteil, bei der das Lanzettenteil an seinem einen Ende mit einem Knopfteil und an seinem anderen Ende mit einem vorstehenden Schneidenabschnitt einer Lanzette versehen ist, bei der ferner das Buchsenteil zur Aufnahme und zur axialen Führung des Lanzettenteils ausgebildet und an seinem einen Ende mit einem Flansch versehen ist, der in einer ersten Gebrauchsstellung der Vorrichtung mittels eines mechanisch überwindbaren Anschlages in einem ersten Abstand von dem Knopfteil angeordnet ist, derart, daß sich der Schneidenabschnitt innerhalb des Buchsenteils befindet, und wobei das Knopfteil und der Flansch unter Überwindung des Anschlages schlagartig in eine zweite Gebrauchsstellung aneinander mit einem zweiten, geringeren Abstand voneinander überführbar sind, so daß der Schneidenabschnitt aus dem anderen Ende des Buchsenteils vorsteht.

Die Erfindung betrifft ferner ein Verfahren zum Herstellen einer Blutentnahmevorrichtung, insbesondere einer Blutentnahmevorrichtung der vorstehend genannten Art, bei dem im Kunststoff-Spritzgießverfahren ein Lanzettenteil und ein Buchsenteil hergestellt werden, wobei das Lanzettenteil an seinem einen Ende mit einem Knopfteil und an seinem anderen Ende mit einem vorstehenden Schneidenabschnitt einer Lanzette versehen ist, und ferner das Buchsenteil zur Aufnahme und zur axialen Führung des Lanzettenteils ausgebildet ist, bei dem weiterhin an das Lanzettenteil ein Schutzelement angespritzt ist, das den Schneidenabschnitt umhüllt und von diesem durch Fingerkraft lösbar ist, und schließlich das Lanzettenteil mit einem ersten zylindrischen Abschnitt ausgeführt wird, in dem ein Schaftabschnitt der Lanzette umspritzt ist.

Eine Vorrichtung und ein Verfahren der vorstehend genannten Art sind aus der DE-PS 31 11 737 bekannt.

Bei der bekannten Blutentnahmevorrichtung ist das Buchsenteil mit einer Durchgangsbohrung versehen, in die sich radial ein Anschlag erstreckt. Das Lanzettenteil ist demgegenüber mit einer Umfangswulst versehen, so daß das Lanzettenteil in der ersten Gebrauchsstellung mit dem Anschlag in Kontakt steht. Drückt man nun das Lanzettenteil und das Buchsenteil gegeneinander, so wird der mechanische Widerstand überwunden, den der radiale Anschlag in der Bohrung des Buchsenteils der Umfangswulst des Lanzettenteils entgegensetzt. Der auf das Lanzettenteil somit ausgeübte Druck wird plötzlich freigesetzt, so daß der Schneidenabschnitt, der zunächst im Buchsenteil verborgen war, aus dem anderen Ende des Buchsenteils hervorschnellt. Wenn die Blutentnahmevorrichtung zum Beispiel auf die Fingerkuppe eines Patienten aufgesetzt worden war, so dringt der Schneidenabschnitt in die Fingerkuppe ein und es tritt Blut des Patienten aus, das dann für eine nachfolgende Laboruntersuchung oder dergleichen abgenommen werden kann.

Die bekannte Vorrichtung wird in einer Kunststoff-Spritzgießmaschine als einstückiges Bauteil gespritzt. Hierzu ist die Anordnung so getroffen, daß das Lanzettenteil derart an einen Ringflansch des Buchsenteils angespritzt ist, daß sich der Schneidenabschnitt gerade in den Ringflansch des Buchsenteils erstreckt, also von dem Kunststoffmaterial des Ringflansches umspritzt ist. Auf diese Weise wird erreicht, daß der Schneidenabschnitt vor dem Gebrauch der Blutentnahmevorrichtung steril gehalten wird, weil er vollständig von Kunststoff umgeben ist.

Wenn eine Blutentnahme vorgenommen werden soll, muß also zunächst das Lanzettenteil durch Fingerkraft vom Buchsenteil gelöst und damit der Schneidenabschnitt aus dem Ringflansch herausgezogen werden. Das Lanzettenteil muß dann mit dem Buchsenteil zusammengefügt werden, indem ein kreiszylindrischer Abschnitt des Lanzettenteils in die Durchgangsbohrung des Buchsenteils eingesetzt wird, bis die erwähnte Ringwulst in Anlage an den Anschlag kommt.

Blutentnahmevorrichtungen der geschilderten Art werden häufig von Patienten, d.h. medizinischen Laien, selbst verwendet, beispielsweise Diabetikern, die in regelmäßigen Abständen selbst eine Blutkontrolle zu Hause durchführen. Bei diesem Personenkreis kann nicht immer damit gerechnet werden, daß die erforderlichen Handhabungen mit der notwendigen Präzision und Sorgfalt ausgeführt werden.

Die bekannte Vorrichtung ist mit einigen Nachteilen behaftet.

Zum einen ist bei der bekannten Vorrichtung von Nachteil, daß der überwindbare Anschlag in Form einer Umfangswulst in Verbindung mit einem radialen Anschlag voraussetzt, daß die Durchgangsbohrung im Buchsenteil jenseits des radialen Anschlages einen größeren Durchmesser hat, nämlich mindestens den der Ringwulst. Damit ist aber eine exakte Führung des Lanzettenteils im Buchsenteil nicht in jedem Falle gewährleistet.

Weiterhin hat die bekannte Vorrichtung den Nachteil, daß sie lediglich für eine einzige Einstechtiefe des Schneidenabschnitts ausgelegt ist. Bei unterschiedlichen Patienten kann jedoch die Hautdicke im Bereich der Entnahmestelle unterschiedlich sein, so daß bei fest eingestellter Einstechtiefe bei einem ersten Patienten mit relativ dünner Haut eine zu große Verletzung angebracht wird, während bei einem anderen Patienten mit relativ dicker Haut die Einstechtiefe unzureichend sein kann.

Weiterhin ist bei der bekannten Vorrichtung von Nachteil, daß die Handhabung eine gewisse Fingerfertigkeit erfordert, und zwar bereits zur Vorbereitung der Vorrichtung, wenn nämlich das Lanzettenteil vom Buchsenteil abgetrennt und beide Teile zusammengefügt werden müssen. Dabei kann es vorkommen, daß infolge einer Unachtsamkeit das Lanzettenteil herunterfällt und damit der Schneidenabschnitt verschmutzt wird. Auch kann nicht ausgeschlossen werden, daß sich der Patient bereits beim Zusammenfügen der beiden Teile am Schneidenabschnitt verletzt. Auch ist insoweit von Nachteil, daß der Patient bereits beim Zusammenfügen der beiden Teile den Schneidenabschnitt sieht, was psychologisch ungünstige Auswirkungen haben kann.

Der Erfindung liegt daher die Aufgabe zugrunde, eine Blutentnahmevorrichtung der eingangs genannten Art dahingehend weiterzubilden, daß die vorstehend genannten Nachteile vermieden werden. Insbesondere soll die Vorrichtung so weitergebildet werden, daß sie bei exakter Führung der beiden Teile aneinander eine einstellbare und damit wählbare Einstechtiefe gestattet und keine schwierigen Manipulationen vor der Gebrauchsfertigkeit der Vorrichtung erforderlich sind, die eine Verschmutzung des Schneidenabschnitts und eine psychologische Beeinträchtigung des Patienten bewirken könnten.

Diese Aufgabe wird bei einer Blutentnahmevorrichtung der eingangs genannten Art zum einen dadurch gelöst, daß der mechanisch überwindbare Anschlag als Sollbruchstelle ausgebildet ist.

Weiterhin wird die Aufgabe erfindungsgemäß dadurch gelöst, daß zum wahlweisen Einstellen des ersten Abstandes in der ersten Gebrauchsstellung mindestens zwei Abstandshalter vorgesehen sind, die alternativ für das Erreichen der zweiten Gebrauchsstellung vorwählbar sind, wobei die mindestens zwei Abstandshalter in der zweiten Gebrauchsstellung einen unterschiedlichen zweiten Abstand herstellen.

Weiterhin wird die Aufgabe erfindungsgemäß dadurch gelöst, daß das Lanzettenteil mit dem Schutzelement in dem Buchsenteil aufgenommen ist, und daß das Schutzelement aus dem anderen Ende des Buchsenteils vorsteht und dort mit Fingerkraft ergreifbar ist.

Schließlich wird die Aufgabe erfindungsgemäß nach dem eingangs genannten Verfahren noch dadurch gelöst, daß das Schutzelement als zweiter zylindrischer Abschnitt ausgeführt wird, der im wesentlichen mit dem ersten Abschnitt fluchtet, und daß das Buchsenteil und das Lanzettenteil mit dem Schutzelement gleichzeitig, aber voneinander getrennt, in einem gemeinsamen Spritzgießwerkzeug hergestellt und bei der Entnahme aus dem Werkzeug zusammengefügt werden, derart, daß das Lanzettenteil mit dem zweiten Abschnitt in dem Buchsenteil aufgenommen ist, und der zweite Abschnitt aus dem unteren Ende des Buchsenteils vorsteht und dort mit Fingern ergreifbar ist.

Die der Erfindung zugrunde liegende Aufgabe wird auf diese Weise vollkommen gelöst.

Die Maßnahme, den mechanisch überwindbaren Anschlag als Sollbruchstelle auszubilden, hat den Vorteil, daß die Funktionen der Führung und des mechanisch überwindbaren Anschlages voneinander getrennt werden können. Auf diese Weise wird die exakte Führung des Lanzettenteils im Buchsenteil nicht beeinträchtigt. Es kann daher - je nach Anforderung im Einzelfall - diejenige Kraft, die zur mechanischen Überwindung des Anschlages erforderlich ist, auch sehr hoch eingestellt werden, ohne daß dadurch die Führungseigenschaften des Buchsenteils gegenüber dem Lanzettenteil beeinträchtigt werden.

Die Maßnahme, mindestens zwei Abstandshalter vorzusehen, die zwei unterschiedliche zweite Abstände in der zweiten Gebrauchsstellung erzeugen, hat den Vorteil, daß mit einfachen Maßnahmen, je nach der Hautdicke des Patienten, eine unterschiedliche Einstechtiefe des Schneidenabschnittes eingestellt werden kann.

Die Maßnahme, das Schutzelement als Abschnitt auszubilden, der vor der Blutentnahme im wesentlichen vom Buchsenteil umgeben ist, hat zum einen den Vorteil, daß der Schneidenabschnitt vom Patienten überhaupt nicht optisch wahrgenommen werden kann, ehe die Blutentnahme vorgenommen wurde. Zum anderen ergibt sich der Vorteil, daß der Schneidenabschnitt während des gesamten Gebrauches der Vorrichtung bis zur Blutentnahme selbst im Buchsenteil geschützt bleibt und daher auch nicht verschmutzt werden kann. Insbesondere ist ein Lösen der beiden Teile voneinander und ein Ineinanderstecken der beiden Teile nicht erforderlich, weil das Lanzettenteil und das Buchsenteil bereits in ihrer Gebrauchsstellung zueinander vormontiert sind und lediglich das Schutzelement aus dem Buchsenteil herausgezogen werden muß, um die Vorrichtung betriebsbereit zu machen.

Die obengenannten Verfahrensmaßnahmen haben schließlich den Vorteil, daß die gesamte Vorrichtung in einem Arbeitsgang und bereits vollständig vormontiert im Bereich der Spritzgießmaschine hergestellt wird. Die bei der Benutzung der Vorrichtung erforderlichen Handhabungen sind dabei auf das absolute Minimum beschränkt.

Bei einer bevorzugten Ausgestaltung der erfindungsgemäßen Vorrichtung ist die Sollbruchstelle als durchstoßbarer Bodenabschnitt an einem der Teile ausgebildet und an dem anderen Teil ist mindestens ein dem Bodenabschnitt zuordenbarer Zapfen vorgesehen.

Diese Maßnahme hat den Vorteil, daß durch die Dimensionierung des durchstoßbaren Bodenabschnitts die Kraft zum Überwinden des Anschlages in weiten Bereichen einstellbar ist.

Bei einer anderen Variante einer konstruktiven Ausbildung der Sollbruchstelle ist diese als abscherbarer Steg oder als abscherbare Nase ausgebildet, die in entsprechender Weise von einem Vorsprung abgeschert werden können. Darüber hinaus ist es selbstverständlich auch möglich, sonstige abbrechbare oder abscherbare oder abreißbare Elemente als Sollbruchstelle vorzusehen.

Bei einer weiteren bevorzugten Ausführungsform der Erfindung stellen die mindestens zwei Abstandshalter auch in der ersten Gebrauchsstellung einen unterschiedlichen ersten Abstand her.

Diese Maßnahme hat den Vorteil, daß der Weg zwischen der ersten und der zweiten Gebrauchsstellung und damit der Schwung eingestellt werden können, mit der der Schneidenabschnitt aus dem Buchsenteil herausschnellt. Es kann daher eine Differenzierung nach weicher und harter Haut des Patienten vorgenommen werden.

Bei einer weiteren bevorzugten Ausgestaltung der Erfindung sind die Abstandshalter als Zapfen ausgebildet.

Diese Maßnahme hat den Vorteil, daß eine besonders einfache Konstruktion entsteht und die Teile der Vorrichtung auf einfache Weise mittels einer Spritzgießmaschine hergestellt werden können.

In diesem Falle ist weiterhin besonders bevorzugt, wenn die Zapfen beim Übergang von der ersten in die zweite Gebrauchsstellung in einer Bohrung laufen, wobei mindestens einer der Zapfen einen verdickten Abschnitt aufweist, dessen Durchmesser größer ist als der Durchmesser der Bohrung.

Diese Maßnahme hat den Vorteil, daß eine zusätzliche Führung des Lanzettenteils am Buchsenteil stattfindet, weil die Zapfen in der genannten Bohrung gleiten. Außerdem ergibt sich eine besonders einfache Gestaltung des Anschlages zur Erzeugung unterschiedlicher zweiter Abstände, weil die Zapfen in weiten Bereichen mit verdickten Abschnitten unterschiedlicher axialer Länge ausgerüstet werden können. Auf diese Weise können durch Vorsehen entsprechend vieler Zapfen zwei, drei oder noch mehr unterschiedlicher Einstechtiefen mit ein- und derselben Blutentnahmevorrichtung einstellbar gemacht werden.

Bei einer Weiterbildung dieser Ausführungsbeispiele sind die Zapfen an dem Knopfteil vorgesehen und in dem Ringflansch sind Durchgänge für die Zapfen angeordnet.

Diese Maßnahme hat den Vorteil, daß die unterschiedlichen Stellungen, d.h. Zuordnungen der mehreren Zapfen zu der einen Bohrung durch einfaches Verdrehen vom Lanzettenteil relativ zum Buchsenteil eingestellt werden können. Durch entsprechende Anschläge oder elastische Verrastungen in Drehrichtung können diese Stellungen entsprechend mechanisch fixiert und durch geeignete optische Markierungen auch angezeigt werden.

Bei diesen Ausführungsbeispielen ist ferner bevorzugt, wenn die Zapfen eine unterschiedliche Länge aufweisen.

Diese Maßnahme hat den Vorteil, daß auf besonders einfache konstruktive Weise der bereits erwähnte unterschiedliche erste Abstand hergestellt werden kann.

Besonders bevorzugt ist in diesem Zusammenhang, wenn einer der Zapfen an einem der Teile angeordnet ist und in der ersten Gebrauchsstellung auf einer als durchstoßbarer Bodenabschnitt ausgebildeten Sollbruchstelle an dem anderen Teil anliegt.

Diese Maßnahme kombiniert die bereits erwähnten Vorteile der Zapfen als Abstandshalter einerseits mit dem durchstoßbaren Bodenabschnitt andererseits. Der durchstoßbare Bodenabschnitt ist in diesem Falle bevorzugt am Boden einer Sackbohrung im Ringflansch angeordnet.

Bei weiteren Ausführungsformen der Erfindung, bei denen das Lanzettenteil in an sich bekannter Weise einen ersten zylindrischen Abschnitt umfaßt, in dem ein Schaftabschnitt der Lanzette gehalten ist, ist darüber hinaus das Schutzelement als zweiter zylindrischer Abschnitt ausgebildet, der im wesentlichen mit dem ersten Abschnitt fluchtet.

Diese Maßnahmen haben den Vorteil, daß das Lanzettenteil insgesamt, d.h. zusammen mit dem Schutzelement, in das Buchsenteil einschiebbar ist, mit der Folge, daß der Schneidenabschnitt ständig geschützt ist und das Schutzelement in einfacher Weise von dem Lanzettenteil gelöst werden kann. Sollte sich in der Zeit vor der Benutzung in der Kammer der Durchgangsbohrung Schmutz ansammeln, so wird eine Kontamination der Nadel infolge der beiden miteinander fluchtenden Abschnitte sicher verhindert, da das Schutzelement erst kurz vor Gebrauch entfernt wird.

Hierzu ist besonders bevorzugt, wenn mindestens einer der Abschnitte mit einer Spitze versehen ist und die Spitze den Übergang zu dem jeweils anderen Abschnitt bildet.

Diese Maßnahme hat den Vorteil, daß die Materialbrücke zwischen den Abschnitten beliebig klein gemacht werden kann, so daß zwar einerseits der Schneidenabschnitt steril umschlossen ist, andererseits aber das Schutzelement mit geringer Kraft vom Schneidenabschnitt lösbar ist.

Bei all diesen Ausführungsbeispielen ist besonders bevorzugt, wenn sämtliche Elemente, mit Ausnahme der Lanzette, aus Kunststoff bestehen.

Ferner ist bei allen Ausführungsbeispielen bevorzugt, wenn das Buchsenteil und das Lanzettenteil in Klemmpassung hergestellt werden oder eine Anordnung mit einem Widerhaken verwendet wird.

Diese Maßnahmen haben den Vorteil, daß die beiden Teile im zusammengesteckten Zustand unverlierbar miteinander verbunden sind.

Weitere Vorteile ergeben sich aus der Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1a und 1b: eine perspektivische Ansicht, einmal von schräg oben und einmal von schräg unten (abschnittsweise) eines Lanzettenteils eines Ausführungsbeispiels einer erfindungsgemäßen Blutentnahmevorrichtung;
- Fig. 2: in einer Darstellung, ähnlich Fig. 1a, eine perspektivische Ansicht eines zugehörigen Buchsenteils;
- Fig. 3a und 3b: eine Seitenansicht, teilweise im Schnitt, sowie eine Frontansicht des Buchsenteils gemäß Fig. 2;
- Fig. 4a und 4b: Darstellungen, ähnlich Fig. 3a und 3b, des Lanzettenteils gemäß Fig. 1a und 1b;
- Fig. 5: eine Seitenansicht, teilweise im Schnitt, durch die Blutentnahmevorrichtung bei zusammengestecktem Lanzettenteil und Buchsenteil, vor der Gebrauchsfertigkeit der Vorrichtung;
- Fig. 6: eine Darstellung, ähnlich Fig. 5, jedoch in einer ersten Gebrauchsstellung der Vorrichtung;
- Fig. 7: eine Darstellung, ähnlich Fig. 6, jedoch in einer zweiten Gebrauchsstellung der Vorrichtung;
- Fig. 8: eine Darstellung, ähnlich Fig. 7, jedoch bei einer veränderten Einstellung der Vorrichtung mit geringerer Einstechtiefe;
- Fig. 9: eine Draufsicht, ähnlich Fig. 3b auf ein Knopfteil, gemäß einem weiteren Ausführungsbeispiel der Erfindung; und
- Fig. 10: eine Darstellung, ähnlich Fig. 6, jedoch für ein weiteres Ausführungsbeispiel der Erfindung.

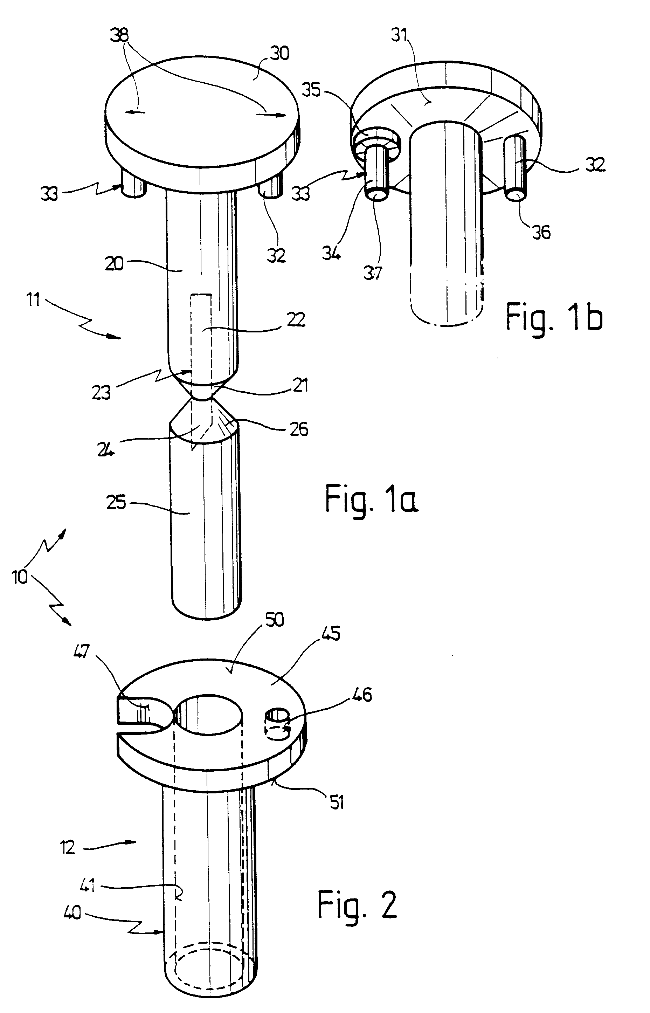

In den Fig. 1 - 4 bezeichnet 10 insgesamt eine Blutentnahmevorrichtung, wie sie auch zum Eigengebrauch von Patienten eingesetzt werden. Mit der Blutentnahmevorrichtung 10 soll aus einem geeigneten Körperteil, insbesondere einer Fingerkuppe, des Patienten eine Blutprobe entnommen werden, wobei die Entnahme einiger Blutstropfen ausreicht.

Die Blutentnahmevorrichtung 10 umfaßt ein Lanzettenteil 11 und ein Buchsenteil 12.

Das Lanzettenteil 11 weist einen ersten zylindrischen Abschnitt 20 auf, der in Fig. 1a unten in eine konische Spitze 21 ausläuft. Der erste Abschnitt 20 umschließt einen Schaftabschnitt 22 einer Lanzette 23, deren Schneideabschnitt 24 somit über den ersten Abschnitt 20 axial vorsteht. Ein zweiter zylindrischer Abschnitt 25 des Lanzettenteils 11 umschließt den Schneidenabschnitt 24. Der zweite Abschnitt 25 läuft in der Darstellung der Fig. 1 nach oben ebenfalls in eine konische Spitze 26 aus.

Wenn das Lanzettenteil 11 einstückig aus Kunststoff gespritzt ist, verbleibt im Übergang zwischen den Spitzen 21 und 26 nur eine sehr dünne Materialbrücke, die den Schneidenabschnitt 24 der Lanzette 23 umschließt.

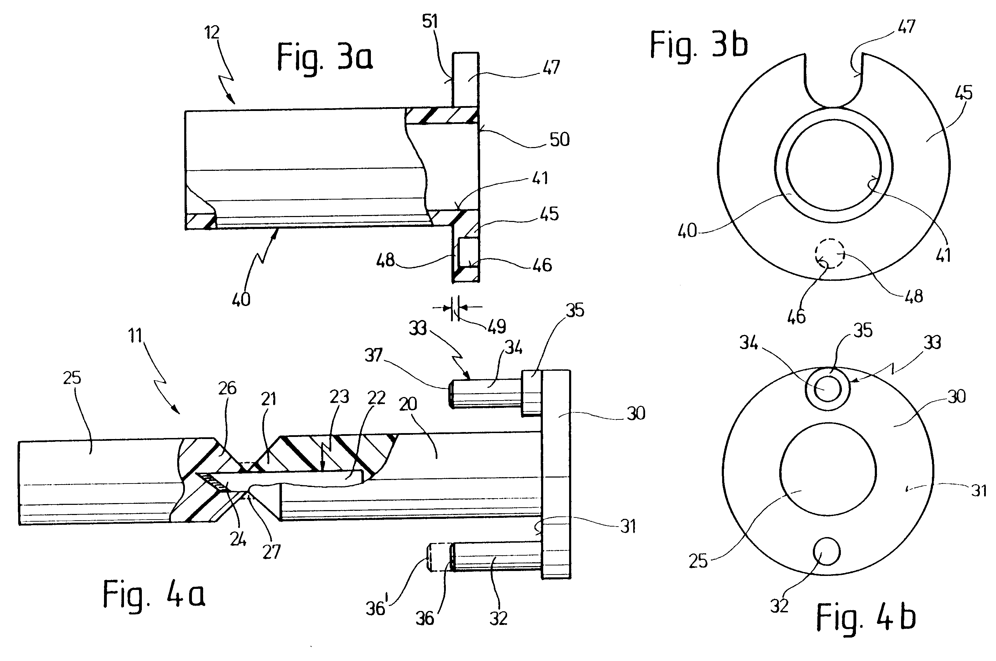

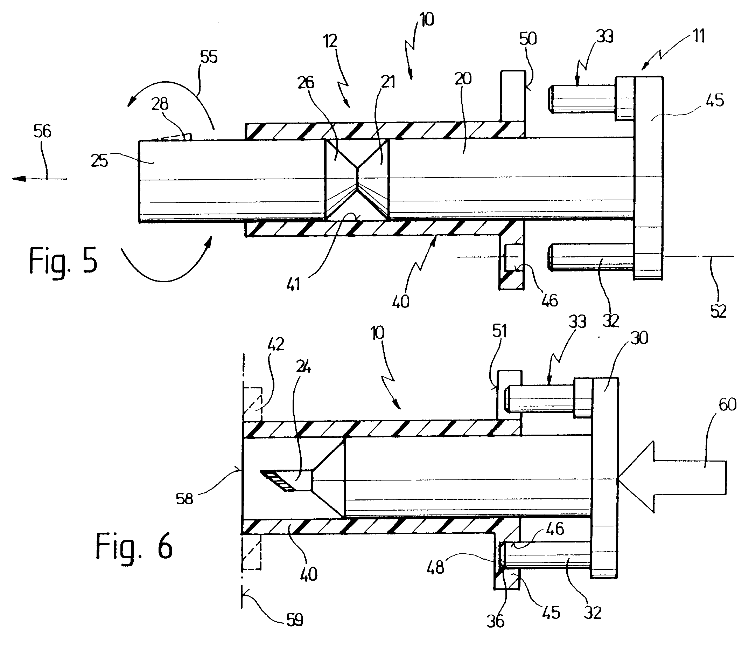

Wie Fig. 4a zeigt, kann an dieser Stelle auch ein zylindrischer Übergang 27 (dort gestrichelt und unmaßstäblich dargestellt) vorgesehen werden, um den sterilen Einschluß der Lanzette 23 zu garantieren. Am freien Ende des zweiten zylindrischen Abschnittes 25 kann ein elastischer Widerhaken 28 (vergleiche Fig. 5) vorgesehen werden, um das Lanzettenteil 11 nach dem Einstecken in das Buchsenteil 12 unverlierbar zu machen.

An das in Fig. 1a obere Ende des ersten Abschnitts 20 ist ein Knopfteil 30 angeformt, das ebenfalls zylindrisch ausgebildet ist, jedoch axial wesentlich kürzer und im Durchmesser wesentlich größer als die Abschnitte 20 und 25 ist.

An der Unterseite 31 des Knopfteils 30 sind im dargestellten Ausführungsbeispiel ein erster Zapfen 32 sowie um 180° in Umfangsrichtung versetzt ein zweiter Zapfen 33 auf demselben Umfang angeformt. Es versteht sich, daß bei Bedarf auch drei, vier oder noch mehr Zapfen dieser Art dort vorgesehen sein können.

Während der erste Zapfen 32 axial gleichförmig dünn durchgeht, ist der zweite Zapfen 33 mit zwei axialen Abschnitten ausgestattet, nämlich einem unteren dünnen Abschnitt 34 und einem oberen verdickten Abschnitt 35. Beide Zapfen 32, 33 können an ihrem freien Ende mit einer Spitze 36 bzw. 37 versehen sein, beispielsweise einem kleinen Konus.

In Fig. 1a ist ferner zu erkennen, daß auf der gegenüberliegenden Seite des Knopfteils 30 an der Position der Zapfen 32, 33 Markierungen 38 angebracht sein können, die weiter unten noch erläutert werden.

Das Buchsenteil 12 umfaßt einen hohlzylindrischen Abschnitt 40, der von einer Durchgangsbohrung 41 durchdrungen ist. Der Innendurchmesser der Durchgangsbohrung 40 entspricht dabei dem gleichen Außendurchmesser der zylindrischen Abschnitte 20 und 25, wobei die Anordnung vorzugsweise so getroffen ist, daß der Innendurchmesser der Durchgangsbohrung 41 geringfügig geringer bemessen ist, um eine Klemmpassung zwischen den Abschnitten 20, 25 des Lanzettenteils 11 und der Durchgangsbohrung 41 des Buchsenteils 12 herzustellen, wenn nicht ein Widerhaken 28, wie oben beschrieben, vorgesehen ist.

Der hohlzylindrische Abschnitt 40 kann, wie Fig. 6 zeigt, an seinem dort links dargestellten Ende mit einem Aufsetzflansch 42 versehen sein, um die Aufsetzoberfläche der Blutentnahmevorrichtung 10 am Patienten zu vergrößern.

Der hohlzylindrische Abschnitt 40 ist ferner an seinem in Fig. 2 oberen Ende mit einem Ringflansch 45 versehen, der axial wesentlich kürzer, dafür im Durchmesser aber wesentlich größer als der hohlzylindrische Abschnitt 40 ausgebildet ist. Die Außenabmessungen des Ringflansches 45 können beispielsweise in etwa denjenigen des Knopfteils 30 entsprechen.

Der Ringflansch 45 ist an einer Umfangsposition mit einer Sackbohrung 46 und an der um 180° in Umfangsrichtung versetzten Position mit einer Aussparung 47 versehen. Die Aussparung 47 ist größer bemessen als der verdickte Abschnitt 35 des zweiten Zapfens 33. Die Sackbohrung 46 weist an ihrem Grund einen dünnen Boden 48 auf, wie besonders deutlich in Fig. 3a zu erkennen ist. Die Dicke 49 des Bodens 48 wird, wie weiter unten noch erläutert werden wird, in Abhängigkeit von der gewünschten Durchstoßkraft und von der Festigkeit des Materials, aus dem der Ringflansch 45 besteht, vorgegeben.

Mit 50 ist die Oberseite und mit 51 die Unterseite des Ringflansches 45 bezeichnet.

Wie bereits erwähnt wurde, bestehen das Lanzettenteil 11 und das Buchsenteil 12 vorzugsweise, mit Ausnahme der Lanzette 23, vollständig aus Kunststoff. Hierzu werden das Lanzettenteil 11 und das Buchsenteil 12 in einer Kunststoff-Spritzgießmaschine hergestellt. Hierzu wird ein Spritzgießwerkzeug verwendet, in dem das Lanzettenteil 11 und das Buchsenteil 12 nebeneinander in einem Spritzvorgang erzeugt werden. Bei der Erzeugung des Lanzettenteils 11 wird dabei zugleich die Lanzette 23 umspritzt, wie dies besonders deutlich in Fig. 4a zu erkennen ist.

Bei dem Ausformen der Teile 11, 12, d.h. der Entnahme der Teile 11, 12 aus dem Spritzgießwerkzeug, werden die Teile 11, 12 nun mittels einer geeigneten Handhabungsvorrichtung (nicht dargestellt) zeitgleich entnommen und während desselben Arbeitsvorganges ineinandergesteckt, indem das Lanzettenteii 11 mit den zylindrischen Abschnitten 20 und 25 in die Durchgangsbohrung 41 des Buchsenteils 12 eingesteckt werden.

Das Ergebnis dieses Entnehmens, Handhabens und Fügens ist in Fig. 5 dargestellt.

Man erkennt, daß die Teile 11, 12 so zusammengesteckt sind, daß die Zapfen 32, 33 zur Oberfläche 50 des Ringflansches 45 zeigen. Der zweite zylindrische Abschnitt 25 ragt über das gegenüberliegende Ende des hohlzylindrischen Abschnittes 40 heraus.

In dieser Position kann der Benutzer der Blutentnahmevorrichtung 10 mit der einen Hand den Ringflansch 45 und mit der anderen Hand das vorstehende Ende des zweiten zylindrischen Abschnittes 25 erfassen. Wenn nun diese beiden Elemente gegeneinander verdreht werden, wie mit einem Pfeil 55 in Fig. 5 angedeutet, so wird der zweite zylindrische Abschnitt 25 vom ersten zylindrischen Abschnitt 20 in Richtung des Pfeiles 56 getrennt, was insbesondere wegen der sehr kleinen Materialbrücke zwischen den Spitzen 21, 26 leicht möglich ist. Damit liegt der Schneidenabschnitt 25, der bis dahin vom zweiten zylindrischen Abschnitt 25 umschlossen und damit steril gehalten war, frei.

Der Benutzer der Blutentnahmevorrichtung 10 kann nun das Lanzettenteil 11 gegenüber dem Buchsenteil 12 verdrehen, bis einer der Zapfen, beispielsweise der Zapfen 32 in Flucht mit der Sackbohrung 46 steht, wie mit einer gemeinsamen Achse 52 in Fig. 5 angedeutet. Die Auswahl, welcher der beiden Zapfen 32 oder 33 in Flucht mit der Sackbohrung 46 gebracht werden soll, wird dem Benutzer durch die bereits erwähnten Markierungen 38 auf der Oberseite des Knopfteiles 30 erleichtert. Die Markierungen 38 können dabei beliebige Form, Gestalt oder Farbe haben und mit entsprechenden Gegenmarkierungen am Ringflansch 45 zusammenwirken, die dort beispielsweise auf dem Umfang in radialer Verlängerung der Position der Sackbohrung 46 angebracht sind.

Die Teile 11, 12 werden nun vom Benutzer in axialer Richtung so zusammengeschoben, daß in der Darstellung der Fig. 6 der erste Zapfen 32 mit seinem freien Ende in die Sackbohrung 46 eintaucht und mit seiner Spitze 36 auf dem Boden 48 aufliegt.

Wie man aus Fig. 6 deutlich erkennen kann, befindet sich der jetzt freiliegende Schneidenabschnitt 24 innerhalb des hohlzylindrischen Abschnittes 40, und zwar in einem Abstand von einer Unterkante 58 des hohlzylindrischen Abschnittes 40, die dem Ringflansch 45 gegenüberliegt. In dieser Konfiguration kann die Vorrichtung 10 auf eine Hautoberfläche des Patienten, beispielsweise eine Fingerkuppe, aufgesetzt werden, die in Fig. 6 mit 59 angedeutet ist.

Der Benutzer der Vorrichtung umfaßt nun, beispielsweise mit seinem Zeige- und Mittelfinger, den hohlzylindrischen Abschnitt 40 an der Unterseite 51 des Ringflansches 45 und drückt mit dem Daumen auf die Oberseite des Knopfteiles 30, wie mit einem Pfeil 60 in Fig. 6 angedeutet. Durch eine geeignete Formgebung der Unterseite 51 kann dabei erreicht werden, daß sich die Finger des Benutzers nicht unterhalb des Bodens 48 befinden.

Wird nun die Kraft zwischen den Fingern (Pfeil 60) erhöht, so durchbricht schließlich der erste Zapfen 32 den Boden 48 der Sackbohrung 46, so daß das Lanzettenteil 11 in der Darstellung der Fig. 6 nach links schnellt und damit der Schneidenabschnitt 24 über die Unterkante 58 hinausfährt.

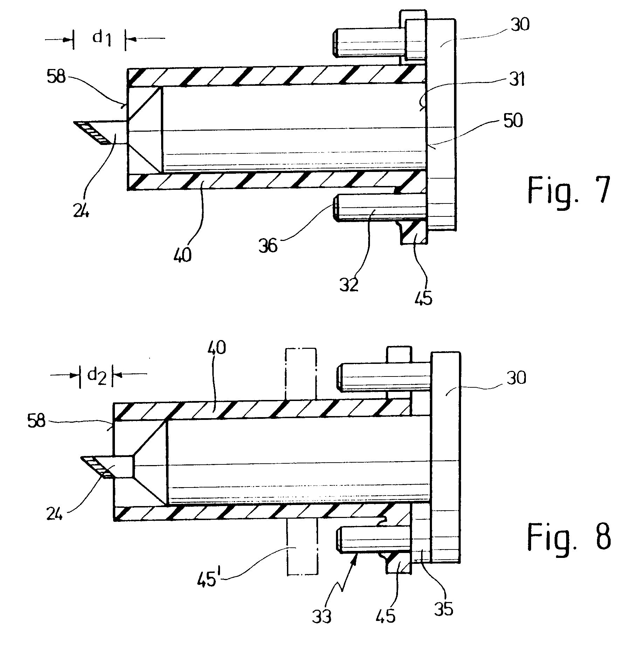

Die erreichte Endstellung ist in Fig. 7 dargestellt. Man erkennt, daß der erste Zapfen 32 den Boden 48 durchstoßen hat und daß das Knopfteil 30 mit seiner Unterseite 31 auf der Oberseite 50 des Ringflansches 45 aufliegt.

Die Anordnung, d.h. die Dimensionierung der diversen axialen Längen, ist dabei so getroffen, daß der Schneidenabschnitt 24 in dieser Endstellung der Fig. 7 um einen vorbestimmten Betrag d₁ über die Unterkante 58 des hohlzylindrischen Abschnittes 40 vorsteht. Dieser Betrag d₁ entspricht der Einstechtiefe.

Wenn hingegen eine Position ähnlich Fig. 6 in der Weise eingestellt wird, daß statt des ersten Zapfens 32 der zweite Zapfen 33 in Eingriff mit der Sackbohrung 46 gebracht wird, so stellt sich nach dem Durchbohren des Bodens 48 eine Endposition gemäß Fig. 8 ein, bei der das Knopfteil 30 und der Ringflansch 45 noch einen axialen Abstand zueinander einnehmen, der der axialen Abmessung des verdickten Abschnittes 35 am zweiten Zapfen 33 entspricht.

In diesem Falle ist die Einstechtiefe auf den Betrag d₂ vermindert.

Man kann also durch Verdrehen des Lanzettenteils 11 relativ zum Buchsenteil 12 und das Ineingriffbringen von entweder dem ersten Zapfen 32 oder dem zweiten Zapfen 33 mit der Sackbohrung 46 die Einstechtiefe mit d₁ oder d₂ unterschiedlich groß einstellen. Hierauf deuten auch die Markierungen 38 hin, die beispielsweise in Form unterschiedlich langer Pfeile ausgeführt sind.

Es versteht sich dabei, daß statt zwei Zapfen 32 und 33 auch drei oder mehr Zapfen vorgesehen werden können, um eine größere Abstufung von Einstechtiefen zu erreichen.

Als erste Variante des vorstehend beschriebenen Ausführungsbeispiels ist in Fig. 4a mit 36' angedeutet, daß die axiale Länge der Zapfen unterschiedlich eingestellt werden kann. Der Betrag der Einstechtiefe d₁ bzw. d₂ in Fig. 7 bzw. Fig. 8 ist unabhängig von der axialen Länge der Zapfen 32 bzw. 33. Ein längerer Zapfen 32 oder 33 hat jedoch einen längeren "Anlauf" zwischen der ersten Gebrauchsstellung gemäß Fig. 6 und der zweiten Gebrauchsstellung gemäß Fig. 7 bzw. 8 zur Folge. Die Wucht des Aufpralls der beiden Teile 11, 12 ist daher bei entsprechend längerem Zapfen auch entsprechend größer. Man kann daher für weichere und härtere Haut dementsprechend größere oder kleinere Auftreffkräfte einstellen.

Ferner ist in Fig. 8 noch eine andere Variante dargestellt, bei der am hohlzylindrischen Abschnitt 40 im Abstand vom Ringflansch 45 noch ein Hilfsflansch 45' angeformt ist, mit dem die Anordnung besser ergriffen werden kann, ohne daß die Finger des Benutzers mit dem vorschnellenden Zapfen 33 in Kontakt geraten.

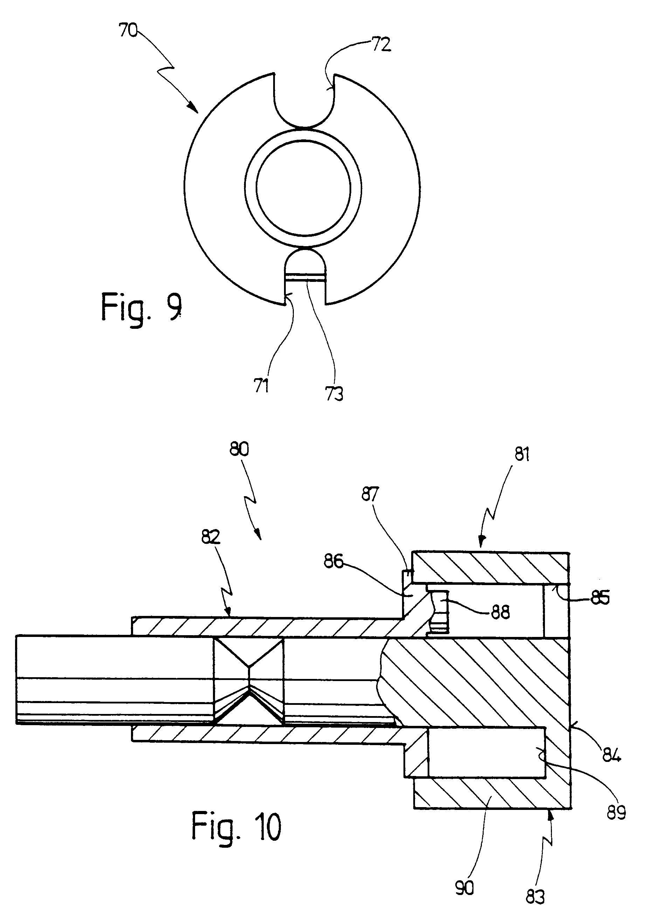

Fig. 9 zeigt in einer Darstellung ähnlich Fig. 3b eine weitere konstruktive Variante für eine Sollbruchstelle.

Hierzu ist ein Knopfteil 70 an derjenigen Stelle, an dem sich bei dem zuvor beschriebenen Ausführungsbeispiel die Sackbohrung 46 mit dem Boden 48 befand, eine erste Aussparung 71 vorgesehen, die sich wiederum diametral gegenüber einer zweiten Aussparung 72 befindet. Die zweite Aussparung 72 kann ebenso beschaffen sein wie die Ausparung 47 im zuvor beschriebenen Ausführungsbeispiel.

In der ersten Ausparung 71 ist ein dünner Steg 73 ausgebildet, bspw. an dem vom Betrachter der Fig. 9 abgewandten Seite des Knopfteils 70 oder auch auf halber Höhe der ersten Aussparung 71.

Der Steg 73 ist in seiner Breite und Tiefe so bemessen, daß er wiederum als Sollbruchstelle wirkt, ähnlich wie der Boden 48 in der Sackbohrung 46 bei dem zuvor beschriebenen Ausführungsbeispiel.

Zum Durchbrechen oder Abscheren des Steges 73 muß am (nicht dargestellten) Lanzettenteil ein entsprechender Vorsprung vorgesehen werden, der zweckmäßigerweise durch die erste Aussparung 71 geführt wird.

Fig. 10 ist in einer Darstellung ähnlich Fig. 6 ein weiteres Ausführungsbeispiel der Erfindung dargestellt.

Bei diesem Ausführungsbeispiel weist eine Blutentnahmevorrichtung 80 wiederum ein Lanzettenteil 81 und ein Buchsenteil 82 auf. Am Lanzettenteil 81 ist am in Fig. 10 rechten Ende wiederum ein Knopfteil 83 angeformt, das in diesem Falle jedoch nach Art einer Kappe ausgebildet ist.

In einer oberen Oberfläche 84 des Knopfteils 83 ist an der in Fig. 10 oberen Umfangsposition eine Bohrung 85 angebracht.

Das Knopfteil 83 umschließt kappenartig einen Ringflansch 86 des Buchsenteils 82. Lediglich an der in Fig. 10 oberen Umfangsposition ist der Ringflansch 86 mit einer vorspringenden Nase 87 versehen, auf der das Knopfteil 83 in axialer Richtung aufsitzt.

Die Nase 87 dient in diesem Falle als Sollbruchstelle, da sie vom kappenartigen Knopfteil 83, und zwar dessen Wand 90, abgeschert werden kann, wenn das Lanzettenteil 81 gegen das Buchsenteil 82 in axialer Richtung gedrückt wird.

Um auch in diesem Falle eine unterschiedliche Einstechtiefe zu erreichen, kann auf der Oberseite des Ringflansches 86 ein Zapfen 88 angeordnet sein. Der Durchmesser des Zapfens 88 ist kleiner als der der Bohrung 85.

In der in Fig. 10 dargestellten Stellung wird das Lanzettenteil 81 gegenüber dem Buchsenteil 82 die volle Einstechdistanz durchmessen, weil im zusammengedrückten Zustand der Zapfen 88 durch die Bohrung 85 hindurchtritt.

Wird hingegen zum Einstellen einer kleineren Einstechtiefe das Buchsenteil 82 aus der in Fig. 10 dargestellten Stellung um 180° in Umfangsrichtung verdreht, so daß sich die Bohrung 85 nunmehr unten befindet, so wird der Zapfen 88 auf eine Innenfläche 89 des Knopfteils 83 auftreffen und damit die Einstechtiefe um die axiale Länge des Zapfens 88 vermindert sein.

Die Ausführungsbeispiele der Figuren 9 und 10 sollen somit veranschaulichen, daß die Sollbruchstelle gemäß der vorliegenden Erfindung in mannigfaltiger Weise konstruktiv ausgestaltet werden kann, ohne den Rahmen der vorliegenden Erfindung zu verlassen.

## Patentansprüche

1. Blutentnahmevorrichtung mit einem Lanzettenteil (11; 81) und einem Buchsenteil (12; 82), bei der das das Lanzettenteil (11; 81) an seinem einen Ende mit einem Knopfteil (30; 70; 83) und an seinem anderen Ende mit einem vorstehenden Schneidenabschnitt (24) einer Lanzette (23) versehen ist, bei der ferner das Buchsenteil (12; 82) zur Aufnahme und zur axialen Führung des Lanzettenteils (11; 81) ausgebildet und an seinem einen Ende mit einem Flansch (45; 86) versehen ist, der in einer ersten Gebrauchsstellung der Vorrichtung mittels eines mechanisch überwindbaren Anschlages in einem ersten Abstand von dem Knopfteil (30; 70; 83) angeordnet ist, derart, daß sich der Schneidenabschnitt (24) innerhalb des Buchsenteils (12; 82) befindet, und wobei das Knopfteil (30; 70; 83) und der Flansch (45; 86) unter Überwindung des Anschlages schlagartig in eine zweite Gebrauchsstellung aneinander mit einem zweiten, geringeren Abstand voneinander überführbar sind, so daß der Schneidenabschnitt (24) aus dem anderen Ende des Buchsenteils (12; 82) vorsteht, dadurch gekennzeichnet, daß der mechanisch überwindbare Anschlag als Sollbruchstelle ausgebildet ist.

2. Vorrichtung nach Anspruch 1, dadurch gekennzeichnet, daß die Sollbruchstelle als durchstoßbarer Bodenabschnitt (48) an einem der Teile (12; 82) ausgebildet und daß an dem anderen Teil (11; 81) mindestens ein dem Bodenabschnitt (48) zuordenbarer Zapfen (32, 34) vorgesehen ist.

3. Vorrichtung nach Anspruch 1, dadurch gekennzeichnet, daß die Sollbruchstelle als abscherbarer Steg (73) an einem der Teile ausgebildet und daß an dem anderen Teil mindestens ein dem Steg (73) zuordenbarer Vorsprung vorgesehen ist.

4. Vorrichtung nach Anspruch 1, dadurch gekennzeichnet, daß die Sollbruchstelle als abscherbare Nase (87) an einem der Teile ausgebildet und daß an dem anderen Teil mindestens ein der Nase (87) zuordenbarer Vorsprung vorgesehen ist.

5. Vorrichtung nach einem oder mehreren der Ansprüche 1 bis 4, dadurch gekennzeichnet, daß das Lanzettenteil (11; 81) und das Buchsenteil (12; 82) jeweils einstückig aus Kunststoff bestehen.

6. Blutentnahmevorrichtung, insbesondere nach einem oder mehreren der Ansprüche 1 - 5, mit einem Lanzettenteil (11; 81) und einem Buchsenteil (12; 82), bei der das Lanzettenteil (11; 81) an seinem einen Ende mit einem Knopfteil (30; 83) und an seinem anderen Ende mit einem vorstehenden Schneidenabschnitt (24) einer Lanzette (23) versehen ist, bei der ferner das Buchsenteil (12; 82) zur Aufnahme und zur axialen Führung des Lanzettenteils (11; 81) ausgebildet und an seinem einen Ende mit einem Flansch (45; 86) versehen ist, der in einer ersten Gebrauchsstellung der Vorrichtung mittels eines mechanisch überwindbaren Anschlages in einem ersten Abstand von dem Knopfteil (30; 83) angeordnet ist, derart, daß sich der Schneidenabschnitt (24) innerhalb des Buchsenteils (12; 82) befindet, und wobei das Knopfteil (30; 83) und der Flansch (45; 86) unter Überwindung des Anschlages schlagartig in eine zweite Gebrauchsstellung aneinander mit einem zweiten, geringeren Abstand voneinander überführbar sind, so daß der Schneidenabschnitt (24) aus dem anderen Ende des Buchsenteils (12; 82) vorsteht, dadurch gekennzeichnet, daß zum wahlweisen Einstellen des ersten Abstandes in der ersten Gebrauchsstellung mindestens zwei Abstandshalter vorgesehen sind, die alternativ für das Erreichen der zweiten Gebrauchsstellung vorwählbar sind, wobei die mindestens zwei Abstandshalter in der zweiten Gebrauchsstellung einen unterschiedlichen zweiten Abstand herstellen.

7. Vorrichtung nach Anspruch 6, dadurch gekennzeichnet, daß die mindestens zwei Abstandshalter in der ersten Gebrauchsstellung einen unterschiedlichen ersten Abstand herstellen.

8. Vorrichtung nach Anspruch 6 oder 7, dadurch gekennzeichnet, daß die Abstandshalter als Zapfen (32, 33; 88) ausgebildet sind.

9. Vorrichtung nach Anspruch 8, dadurch gekennzeichnet, daß die Zapfen (32, 33) beim Übergang von der ersten in die zweite Gebrauchsstellung in einer Bohrung (46) laufen, wobei mindestens einer der Zapfen (33) einen verdickten Abschnitt (35) aufweist, dessen Durchmesser größer ist als der Durchmesser der Bohrung (46).

10. Vorrichtung nach Anspruch 8 oder 9, dadurch gekennzeichnet, daß die Zapfen (32, 33) an dem Knopfteil (30) vorgesehen und daß in dem Flansch (45) Durchgänge (46, 47) für die Zapfen (32, 33) angeordnet sind.

11. Vorrichtung nach einem oder mehreren der Ansprüche 8 - 10, dadurch gekennzeichnet, daß die Zapfen (32, 33) eine unterschiedliche Länge (36') aufweisen.

12. Vorrichtung nach einem oder mehreren der Ansprüche 8 - 11, dadurch gekennzeichnet, daß einer der Zapfen (32, 33) an einem der Teile (12) angeordnet ist und in der ersten Gebrauchsstellung auf einer als durchstoßbarer Bodenabschnitt (48) ausgebildeten Sollbruchstelle an dem anderen Teil (11) anliegt.

13. Blutentnahmevorrichtung, insbesondere nach einem oder mehreren der Ansprüche 1 - 12 mit einem Lanzettenteil (11) und einem Buchsenteil (12), bei der das das Lanzettenteil (11) an seinem einen Ende mit einem Knopfteil (30) und an seinem anderen Ende mit einem vorstehenden Schneidenabschnitt (24) einer Lanzette (23) versehen ist und an das Lanzettenteil (11) ein Schutzelement angesetzt ist, das den Schneidenabschnitt (24) umhüllt und von diesem durch Fingerkraft lösbar ist, bei der ferner das Buchsenteil (12) zur Aufnahme und zur axialen Führung des Lanzettenteils (11) ausgebildet und an seinem einen Ende mit einem Flansch (45) versehen ist, der in einer ersten Gebrauchsstellung der Vorrichtung mittels eines mechanisch überwindbaren Anschlages in einem ersten Abstand von dem Knopfteil (30) angeordnet ist, derart, daß sich der Schneidenabschnitt (24) innerhalb des Buchsenteils (12) befindet, und wobei das Knopfteil (30) und der Flansch (45) unter Überwindung des Anschlages schlagartig in eine zweite Gebrauchsstellung aneinander mit einem zweiten, geringeren Abstand voneinander überführbar sind, so daß der Schneidenabschnitt (24) aus dem anderen Ende des Buchsenteils (12) vorsteht, dadurch gekennzeichnet, daß das Lanzettenteil (12) mit dem Schutzelement in dem Buchsenteil (12) aufgenommen ist, und daß das Schutzelement aus dem anderen Ende des Buchsenteils (12) vorsteht und dort mit Fingern ergreifbar ist.

14. Vorrichtung nach Anspruch 13, dadurch gekennzeichnet, daß das Lanzettenteil (12) einen ersten zylindrischen Abschnitt umfaßt, in dem ein Schaftabschnitt (22) der Lanzette (23) gehalten ist, und daß das Schutzelement als zweiter zylindrischer Abschnitt (25) ausgebildet ist, der im wesentlichen mit dem ersten Abschnitt (20) fluchtet, so daß die Kammer damit abgedichtet ist.

15. Vorrichtung nach Anspruch 14, dadurch gekennzeichnet, daß der ersten und der zweite Abschnitt (20, 25) einstückig sind und aus Kunststoff bestehen.

16. Vorrichtung nach einem oder mehreren der Ansprüche 13 - 15, dadurch gekennzeichnet, daß mindestens einer der Abschnitte (20, 25) mit einer Spitze (21, 26) versehen ist, und daß die Spitze (21, 26) den Übergang zu dem jeweils anderen Abschnitt (20, 25) bildet.

17. Vorrichtung nach Anspruch 16, dadurch gekennzeichnet, daß das Schutzelement mit einem Widerhaken (28) versehen ist, der das in das Buchsenteil (12) einschobene Lanzettenteil (11) unverlierbar macht.

18. Verfahren zum Herstellen einer Blutentnahmevorrichtung, insbesondere einer Blutentnahmevorrichtung nach einem oder mehreren der Ansprüche 1 - 17, bei dem im Kunststoff-Spritzgießverfahren ein Lanzettenteil (11) und ein Buchsenteil (12) hergestellt werden, wobei das Lanzettenteil (11) an seinem einen Ende mit einem Knopfteil (30) und an seinem anderen Ende mit einem vorstehenden Schneidenabschnitt (24) einer Lanzette (23) versehen, und ferner das Buchsenteil (12) zur Aufnahme und zur axialen Führung des Lanzettenteils (11) ausgebildet ist, bei dem weiterhin an das Lanzettenteil (12) ein Schutzelement anspritzt ist, das den Schneidenabschnitt (24) umhüllt und von diesem durch Fingerkraft lösbar ist, und schließlich das Lanzettenteil (12) mit einem zylindrischen Abschnitt (20) ausgeführt wird, in dem ein Schaftabschnitt (22) der Lanzette (23) umspritzt ist, dadurch gekennzeichnet, daß das Schutzelement als zweiter zylindrischer Abschnitt (25) ausgeführt wird, der im wesentlichen mit dem ersten Abschnitt (20) fluchtet, und daß das Buchsenteil (12) und das Lanzettenteil (11) mit dem Schutzelement gleichzeitig, aber voneinander getrennt, in einem gemeinsamen Spritzgießwerkzeug hergestellt und bei der Entnahme aus dem Werkzeug zusammengefügt werden, derart, daß das Lanzettenteil (12) mit dem zweiten Abschnitt (25) in dem Buchsenteil (12) aufgenommen ist, und der zweite Abschnitt (25) aus dem anderen Ende des Buchsenteils (12) vorsteht und dort mit Fingern ergreifbar ist.

19. Verfahren nach Anspruch 18, dadurch gekennzeichnet, daß das Lanzettenteil (11) und das Buchsenteil (12) in Klemmpassung hergestellt werden.
